# EUROPÄISCHE PATENTSCHRIFT

(11) **EP 2 190 585 B1**
(45) Veröffentlichungstag und Bekanntmachung des Hinweises auf die Patenterteilung: **11.05.2011**
(21) Anmeldenummer: 08803237.0
(22) Anmeldetag: 26.08.2008
(51) Int. Cl.: B03C 1/28, B03C 1/01

(54) **HOCHGRADIENTENMAGNETSEPARATION BIOLOGISCHEN MATERIALS**
HIGH GRADIENT MAGNET SEPARATION OF BIOLOGICAL MATERIAL
SÉPARATION MAGNÉTIQUE À HAUT GRADIENT DE CHAMP POUR MATÉRIAU BIOLOGIQUE

(30) Priorität: 11.09.2007 DE 102007043281
(43) Veröffentlichungstag der Anmeldung: 02.06.2010
(73) Patentinhaber: X-Zell Biotech Ltd., Bangkok 10110 (TH)
(72) Erfinder: MALASIT, Prida, Bangkok (TH); BHAKDI, Sebastian Chakrit, 35043 Marburg (DE)
(74) Vertreter: Hehl, Ulrich
(86) Internationale Anmeldenummer: PCT/EP2008/061170
(87) Internationale Veröffentlichungsnummer: WO 2009/033947

(56) Entgegenhaltungen:
- WO-A-2006/119308
- DE-A1- 19 516 323
- US-A- 3 826 365
- US-A- 3 983 033
- US-A- 4 664 796
- US-A- 5 200 084
- US-A- 5 466 574
- US-A- 5 512 332
- US-A- 5 536 644
- US-A- 6 013 188

## Beschreibung

Die Erfindung betrifft eine Anwendung der Hochgradientenmagnetseparations (HGMS)-Technologie zur Trennung und Aufreinigung von biologischem Material.

Speziell auf dem Gebiet der medizinisch-biologischen Forschung ist die Abtrennung und Aufreinigung bestimmter Partikel aus heterogenen Partikelsuspensionen für verschiedenste Analyseverfahren von großer Bedeutung. Generell unterscheiden sich die Partikel, welche aufgereinigt werden sollen, im Folgenden "Zielpartikel" genannt, oftmals nur minimal von dem Rest der in der Suspension enthaltenen Partikel, im Folgenden "nicht-Zielpartikel" genannt. Bei Zielpartikeln und nicht-Zielpartikeln handelt es sich oft um Zellen oder Zellfragmente, es können jedoch auch beliebige andere biologische Substanzen sein.

Einige bewährte Abtrennungsverfahren nutzen magnetische Eigenschaften von Zielpartikeln, wobei die Zielpartikel natürliche, "intrinsische" magnetische Eigenschaften besitzen können oder aber vor dem eigentlichen Abtrennungsverfahren durch gezieltes Anheften synthetischer magnetischer Teilchen markiert werden.

Intrinsisch magnetische Partikel sind beispielsweise rote Blutzellen, vorausgesetzt der Fall, dass das in diesen enthaltende Hämoglobin in desoxygeniertem oder oxidiertem, jedoch nicht in oxygeniertem Zustand vorliegt. Hierbei bedeutet desoxygeniert nicht-sauerstofftragend und oxygeniert sauerstofftragend. In letzterem Falle trägt das Hämoglobinmolekül ein Sauerstoffinolekül in nicht kovalenter, also reversibler Bindung. Davon zu unterscheiden ist der oxidierte Zustand des Hämoglobins, in welchem Sauerstoff- oder andere oxidierende Atome kovalent, also nicht reversibel an das zentrale Eisenatom des Hämoglobinmoleküles gebunden sind. Nun tragen die Orbitale des im Hämoglobinmolekül enthaltenen zentralen Eisenatoms sowohl in der desoxygenierten wie in der oxidierten Form (jedoch nicht in der oxygenierten Form) ungepaarte Elektronen, deren ungepaarter Spin die Induktion magnetischer Pole im Eisenatom durch Anlegen eines magnetischen Feldes ermöglicht. Allerdings übt ein angelegtes magnetisches Feld konventioneller Art insgesamt keine gerichtete magnetische Kraft auf ein Partikel aus, welches solche Eisenatome enthält, da sich aufgrund des sehr kleinen Atomdurchmessers anziehende und abstoßende magnetische Kräfte an Nord- und Südpol der polarisierten Eisenatome die Waage halten. Auch wird das Partikel nach Wegnahme des Magnetfeldes seine magnetische Polarisation verlieren. Diese Art des Magnetismus ist als "Paramagnetismus" bekannt. Eine spezielle Form des Paramagnetismus wird gelegentlich auch als "Superparamagnetismus" bezeichnet. Zwischen diesen beiden Formen besteht jedoch physikalisch keine scharfe Trennung, im Folgenden sollen daher die Begriffe "Paramagnetismus" und "paramagnetisch" die Begriffe "Superparamagnetismus" und "superparamagnetisch" mit umschließen.

Auch die gängigen, zur magnetischen Markierung von Zielpartikeln verwandten synthetischen Teilchen sind paramagnetisch und enthalten üblicherweise, ganz ähnlich-dem - oxidierten Hämoglobin, kleinste Mengen oxidierten Eisens oder einer anderen magnetisierbaren Substanz. Sie sind außerdem idealerweise so klein, dass sie in Suspension stabile Kolloide bilden, also auch über lange Zeiträume (Monate bis Jahre) nicht sedimentieren, daher beträgt ihr Durchmesser im Allgemeinen 30-200 nm. Kommerziell erhältliche Teilchen dieser Art werden allein oder an Antikörper gebunden vertrieben, beispielsweise von chemicell GmbH (Eresburgstrasse 22-23, D-12103 Berlin, Deutschland), micromod Partikeltechnologie GmbH (Friedrich-Barnewitz-Str.4, D-18119 Rostock, Deutschland) oder Miltenyi Biotech GmbH (Friedrich Ebert Straße 68, D-51429 Bergisch Gladbach, Deutschland).

Ein bewährtes Verfahren, paramagnetische Partikel aufzureinigen, d.h. von Partikelsuspensionen abzutrennen, ist die Erzeugung extrem hoher magnetischer Feldgradienten. Ein ausreichend hoher magnetischer Feldgradient führt dazu, dass Nord- und Südpole der paramagnetischen Teilchen eine Differenz von An- und Abstoßungskräften und somit insgesamt eine gerichtete magnetische Kraft erfahren. Diese Technologie ist unter der Bezeichnung Hochgradientenmagnetseparation (HGMS) bekannt.

Zu unterscheiden ist hier zwischen sogenannten "internen" und "externen" Hochgradientenmagnetseparatoren. Erstbeschreibungen der HGMS-Technologie bezogen sich auf interne Separatoren. Diese finden sich bei Oberteuffer (IEEE Transactions on Magnetics, Mag-9, No. 3, September 1973:303-306) und in der US 3.676.337. Ein ferromagnetisches Material in einem geeigneten, nicht magnetischen Behältnis, welches als Trennkammer dient, wird in ein starkes, homogenes Magnetfeld eingebracht, welches durch einen Elektromagneten oder durch einen hufeisenförmigen Permanentmagneten (Dipolmagneten) erzeugt werden kann. Das ferromagnetische Material trägt dabei im Allgemeinen die Bezeichnung "Matrix"; es kann filamentös (draht- oder fadenförmig), sphärisch (kugelförmig) oder anderweitig geformt sein, zum Beispiel aus gestanztem Stahlblech bestehen. Das ferromagnetische Material der Matrix erfährt durch das extern angelegte Feld eine Magnetisierung entsprechend seiner magnetischen Suszeptibilität X. Ausgehend von der Oberfläche des Matrixmaterials entstehen dann magnetische Feldgradienten, die über 100 Tesla pro Zentimeter erreichen können, wobei die Größe des Gradienten in umgekehrtem Verhältnis zum Durchmesser der verwandten filamentösen oder sphärischen Elemente steht. "Externe" Hochgradientenmagnetseparatoren erreichen ähnlich hohe Gradienten durch eine technisch aufwendigere, spezielle Anordnung der Magnete außerhalb der eigentlichen Trennkammer, wie beispielsweise offenbart in der WO 98/055236, der WO 99/019071 oder der US 6.241.894 B1. Als wesentlicher Unterschied entfällt hier die Notwendigkeit einer Matrix innerhalb der Trennkammer.

Am weitesten verbreitet in der medizinisch-biologischen Forschung sind heutzutage die internen Hochgradientenmagnetseparatoren. Die US 4.664.796 und die US 5.200.084 beschreiben spezielle Ausführungsarten solcher Separatoren.

Die US 5.200.084 offenbart ein Gerät und ein Verfahren, welches speziell auf die Aufreinigung kleinster Mengen biologischen Materials in den Vertiefungen einer Mikrotiterplatte ausgerichtet ist. Unter anderem werden Aufreinigungen von bis 83% für mit paramagnetischen Partikeln markierte CD4 Zellen aus peripheren mononukleären Blutzellen (PBMCs) berichtet.

Hohe Aufreinigungsraten erzielt derzeit nach dem Wissen der Erfinder allein eine technische Ausführung eines internen Hochgradientenmagnetseparators, wie in der WO 96/26782 und der EP 0.942.766 B1 offenbart. Zur Vermeidung unspezifischer Bindungen von nicht-Zielpartikeln an die Matrix, welche das Aufreinigungsergebnis bei den in den genannten Druckschriften beschriebenen Separatoren beeinträchtigen, besitzt die Trennkammer dieses Separators eine polymerbeschichtete Matrix. Das Polymer wird dabei in mehreren Arbeitsschritten auf die Matrix aufgezogen, wie in der WO 96/26782 ausführlich beschrieben. Die so hergestellte Trennkammer zeichnet sich nach Angaben der Autoren dadurch aus, dass das Polymer auf dem ferromagnetischen Material der Matrix eine feste, geschlossene, flüssigkeits- und ionenundurchlässige und weniger als 1% Wasser enthaltende Beschichtung bildet, und die mit dem Polymer beschichtete Matrix 60-70% des Gesamtvolumens der beschriebenen Trennkammer ausfüllt.

Neben der Verminderung einer unspezifischen Bindung von nicht-Zielpartikeln an die Matrix soll diese gemäß der genannten Veröffentlichung auch eine Schädigung des zu trennenden biologischen Materials durch direkten Kontakt mit dem ferromagnetischen Matrixmaterial vermeiden (physikalische Schädigung), sowie eine chemische Reaktion des ferromagnetischen Matrixmaterials mit der zur Suspension des biologischen Materials verwandten Pufferlösung ausschließen, denn freigesetzte Ionen könnten ebenfalls zur Schädigung des zu trennenden biologischen Materials führen (chemische Schädigung). Diese beiden Arten der Schädigung sind nach dem Wissensstand der Erfinder jedoch als hypothetisch zu betrachten, denn es sind hierzu keine wissenschaftlich gesicherten Erkenntnisse verfügbar. Auch berichteten Paul et al. in Clinical and Laboratory Haematology, 7, 1985:43-53 gegenteilig über keine Beeinträchtigung der Morphologie und Viabilität von Blutzellen und Blutzellfragmenten, welche eine unbehandelte Edelstahlmatrix einer HGMS-Trennsäule passierten.

Diese beschriebene Art Trennkammern sind aufgrund ihres zeitintensiven Herstellungsprozesses vor allem für die Matrixbeschichtung kostspielig. Sie sind kommerziell von Miltenyi Biotech GmbH (loc.cit.) erhältlich.

Hohe Aufreinigungen erzielen sie insbesondere bei Anwendung auf mit synthetischen paramagnetischen Teilchen markierten Partikeln aus Partikelsuspensionen.

Ebenfalls versucht wurde die Anwendung dieser Trennkammer mit beschichteter Matrix auf intrinsisch (natürlich) paramagnetische Partikel. Als intrinsisch paramagnetische Partikel dienten dabei Malaria-infizierte rote Blutzellen. Die Krankheitserreger der Malaria, Parasiten der Gattung Plasmodium aus dem Reich der Protozoen, befallen selektiv rote Blutzellen und haben die Eigenschaft, das zentrale Eisenatom des in der infizierten roten Blutzelle anfallenden freien Hämmoleküls zu dreiwertigem Eisen zu oxidieren. Dieses ist, wie oben beschrieben, paramagnetisch. Daher sollten Malaria-infizierte rote Blutzellen von nicht infizierten und oxygenierten roten Blutzellen in Trennkammern von Hochgradientenmagnetseparatoren abtrennbar sein. Dies wurde erstmals 1981 von Paul et al. gezeigt (Lancet, July 11, 1981:70-71). Die beschriebenen, kommerziell erhältlichen Trennkammern mit beschichteter Matrix sollen heutzutage Aufreinigungen von über 80% erzielen (Uhlemann et al., MACS&more 2000;4 (2):7-8, Trang et al., Malaria Journal 2004;3:1-7).

Diese aufgeführten Untersuchungen beziehen sich jedoch nur auf einen der insgesamt vier bekannten beim Menschen vorkommenden Malariaerreger, nämlich *Plasmodium falciparum,* dem Erreger Malaria tropica, sowie auf einen weiteren Malariaerreger in Nagetieren, *Plasmodium berghei.* Weitere wissenschaftliche Studien zu den insgesamt nahezu 120 weiteren bekannten Plasmodienarten sind nicht verfügbar. Den Erfindern ist allerdings bekannt, dass die Anwendung der kommerziell erhältlichen Trennkammern zur Aufreinigung von roten Blutzellen, welche mit *Plasmodium vivax,* dem Erreger der ebenfalls beim Menschen vorkommenden Malaria tertiana, infiziert sind, nicht immer zu zufriedenstellenden Ergebnissen führte.

Die US5200084 beschreibt ein Verfahren zur Magnetseparation, bei dem beispielsweise Körperflüssigkeiten in einer Pufferlösung kolloidale magnetische Partikel zugesetzt werden. Als Pufferlösung kann eine biokompatible Salzlösung mit 5% bovinem Seralbumin sowie bevorzugt 0,1%-3% Tamol 850 verwendet werden. Die Pufferlösung dient in einem ersten Schritt des Verfahrens dazu, dass sich die magnetischen Partikel in höherem Maße mit den Zielpartikeln verbinden, statt unspezifische Bindungen einzugehen. Nach Abschluss des ersten Schrittes wird die so erhaltene Lösung in eine Trennvorrichtung gegeben, in der Drahtschleifen zur Verstärkung eines äußeren Magnetfeldes vorgesehen sind.

In der US3826365 werden durch Magnettrennung Titanverunreinigungen aus Kaolin entfernt. Dabei wird eine Matrix aus Stahlwolle verwendet.

Aus der US 5536644 ist ein Partikeltrennverfahren zur Trennung einer Substanz von einem flüssigen Medium bekannt. Dazu werden magnetische Partikel unter Bedingungen für eine unspezifische chemische Bindung zugesetzt

Aus den vorangehenden Erläuterungen ist ersichtlich, dass weitere Verbesserungen der Aufreinigungseffektivität der HGMS-Technologie für den Bereich der medizinisch-biologischen Forschung von großem Nutzen wären. Dies gilt auch in Bezug auf die Kosteneffizienz, da die aufwändigen bekannten Trennkammer in verschiedenen Bereichen, besonders aber in der Erforschung von Malaria-Erregerm, die besonders Länder mit kleineren medizinischen und Forschungsetats betrifft, schon allein aus Kostenerwägungen nicht immer verfügbar sind.

Daher ist Aufgabe der Erfindung, eine HGMS-Trennsäule bereitzustellen, welche in kosten günstigerer Weise bessere Aufreinigunasergebnisse erzielt.

Diese Aufgabe wird durch eine HGMS-Trennsäule gemäß Anspruch 1 und ein Verfahren gemäß Anspruch 11 gelöst, wobei interne HGMS eingesetzt wird. Die erfindungsgemäße Lösung beruht auf dem Prinzip, das Problem der unspezifischen Bindung von nicht-Zielpartikeln an die Matrix der HGMS-Trennsäule durch eine Pufferlösung zur Equilibrierung der Trennsäule und zur Suspension des zu trennenden biologischen Materials mit den im Anspruch angegebenen Eigenschaften zu lösen. Auf die aufwändige und kostentreibende Beschichtung kann dann verzichtet werden, weil die unspezifischen Bindungsstellen der Trennsäule durch die Pufferlösung abgesättigt werden. Dabei sind unter unspezifischen Bindungsstellen solche zu verstehen, an denen Partikel sich unabhängig von ihren magnetischen Eigenschaften absetzen, an denen Partikel also mechanisch, elektrisch, chemisch, physikalisch oder noch anders gebunden würden. Damit würden nicht-Zielpartikel unabhängig vom magnetischen Gradienten selektiert, was zu unvollständigen Aufreinigungs- oder Trennungsergebnissen führen würde. Bei dem biologischen Material handelt es sich bevorzugt um Zellen, Zellaggregate oder Zellbestandteile, welche intrinsische paramagnetische Eigenschaften besitzen, oder welche direkt oder indirekt mittels paramagnetischer oder superparamagnetischer Teilchenmagnetisch markierbar sind.

Die erfindungsgemäße Lösung hat den Vorteil, dass die Pufferlösung verhältnismäßig einfach und kostengünstig bereitgestellt werden kann. Eine zeit-, material- und kostenaufwändige Vorbehandlung der Trennsäule und der in ihr enthaltenen Matrix kann entfallen. Dabei hat diese Vereinfachung gegenüber herkömmlichen Trennsäulen mit ihren aufwändigen Beschichtungen der Matrix sogar noch den Vorteil verbesserter Aufreinigungsergebnisse.

Die Pufferlösung weist bevorzugt eine Dichte auf, die der Dichte zu trennender Partikel des biologischen Materials so weitgehend entspricht, dass eine auf die Partikel wirkende Schwerkraft im Wesentlichen kompensiert ist und die Partikel daher in der Pufferlösung nahezu schweben, und/oder die Pufferlösung weist eine hohe Viskosität auf, welche ein laminares Durchströmen der Trennsäule mit für den Trennvorgang geeigneter Fließgeschwindigkeit ermöglicht. Auf diese Weise können die Zielpartikel ohne störenden Schwerkrafteinfluss so lange im Wirkungsbereich des Hochgradientenmagnetfelds gehalten werden, dass sie sich in einem sehr hohen Grad dort absetzen. Somit wird ein besonders hoher Trennungs- oder Aufreinigungsgrad erhalten.

Der Magnet ist vorteilhafterweise ein Permanent- oder Elektromagnet, welcher so geformt ist, dass die Trennsäule in dem von ihm erzeugten insbesondere homogenen Magnetfeld angeordnet werden kann, wobei die Trennsäule durch räumliche Trennung und/oder Abschalten wahlweise unter Einwirkung des Magnetfelds stehen kann oder nicht. Durch die Formpassung kann das Magnetfeld nahe an die Matrix gebracht werden und stellt daher eine hinreichende äußere magnetische Feldstärke bereit. Der Magnet muss darüber hinaus hinreichend stark sein, so dass durch die Bündelung der Feldstärke mittels der Matrix ein starkes Hochgradientenmagnetfeld erzeugt wird. Mittels räumlicher Trennung oder Abschalten können die Zielpartikel dann gezielt festgehalten und freigegeben werden, je nachdem, ob die aus der Trennsäule strömende Flüssigkeit im jeweiligen Arbeitsschritt Zielpartikel enthalten soll oder nicht.

Die Matrix ist vorzugsweise unbeschichtet und/oder weist geordnetes oder ungeordnetes, filamentöses, sphärisches oder anderweitig geformtes Material auf, insbesondere nichtrostenden, magnetischen Edelstahl oder Stahlwolle. Eine unbeschichtete Matrix ist besonders kostengünstig, und eine Beschichtung erfindungsgemäß nicht notwendig. Dennoch wäre alternativ vorstellbar, zusätzlich auch eine Beschichtung vorzusehen, um die Trenn- und Aüfreinigungsergebnisse weiter zu verbessern, wobei diese Beschichtung dann auch unvollständig oder weniger hochwertig sein könnte als herkömmlich. Die relativ weitmaschige Anordnung der Matrix sowie die Verwendung hochwertigen nichtrostenden Edelstahls als Matrixmaterial beugen einer physikalischen oder chemischen Schädigung des zu trennenden biologischen Materials vor, beispielsweise ließen sich so in den unten beschriebenen Versuchen Schädigungen empfindlicher Zellen effektiv vermeiden.

Der Vorratsbehälter ist bevorzugt mit der Trennsäule verbunden, so dass Pufferlösung über eine Zuflussbegrenzungseinrichtung mit einer einstellbaren Fließgeschwindigkeit in die Trennsäule eingebracht werden kann oder nicht, und/oder wobei die Trennsäule eine Durchflussbegrenzungseinrichtung aufweist, welche den Ausfluss aus der und die Strömungsgeschwindigkeit in der Trennsäule beeinflussen kann. Dabei kann die Verbindung zwischen Vorratsbehälter und Trennsäule apparativ, etwa durch eine Rohrleitung, aber ach durch freies Tropfen vorgesehen sein. Die Fließgeschwindigkeit kann damit an das biologische Material, die Pufferlösung und den jeweiligen Arbeitsschritt angepasst werden, wobei auch ein vollständiges Sperren des Zuflusses von Pufferlösung ermöglicht ist, etwa um zum Equilibrieren eine Phase des Ruhens in der Trennsäule vorzusehen, oder in Arbeitsschritten, in denen keine Pufferlösung benötigt wird.

Die Pufferlösung umfasst eine Grundlösung und mindestens ein Makromolekül. Beispielsweise enthält die Pufferlösung von 80 bis 99,8 Gew.-% Grundlösung und von 0,2 bis 20 Gew.-% Makromolekül.

Die Pufferlösung weist dabei vorzugsweise eine Grundlösung auf, deren Ionenstärke in Abhängigkeit von den Makromolekülen so angepasst ist, dass aggregierende Effekte der Makromoleküle auf das biologische Material kompensiert werden, wobei die Grundlösung eine isoosmolare Konzentration aus Kationen Natrium, Kalium, Magnesium oder Kalzium und aus Anionen Chlorid, Phosphat, Sulfat oder Carbonat aufweist, insbesondere eine phosphatgepufferte Kochsalz- oder Sucroselösung oder eine Mischung daraus ist. Die Grundlösung sollte also nach dem unten beschriebenen Mechanismus in ihren Eigenschaften wie pH-Wert und anderen an sowohl das biologische Material wie an die Makromoleküle zum Absättigen der unspezifischen Bindungsstellen angepasst werden, um noch bessere Ergebnisse zu erzielen. Dabei werden unerwünschte Aggregationen des biologischen Materials besonders gut verhindert, wenn die isoosmolare (physiologische) phosphafgepufferte Kochsalzlösung durch isoosmolare phosphatgepufferte Sucroselösung ganz- oder teilweise ersetzt ist. Andere Zuckerlösungen als Sucroselösung sind vorstellbar.

Beispielsweise enthält die Pufferlösung 0,2 bis 10 Gew.-% Gelatine, 9,5-10 Gew.-% Sucrose, 80-95 Gew.-% destilliertes Wasser und Natriumphosphat zur Pufferung, und/oder 3-10% bovines Serumalbumin, 0,85-0,95 Gew.-% Natriumchlorid und 89-98 Gew.-% destilliertes Wasser und Natriumphosphat zur Pufferung und/oder 0,5-20 Gew-% hydrolysiertes Kollagen, 5-10% Gew.-% Sucrose, 0,1-0,9 Gew.-% Natriumchlorid und Natriumphosphat zur Pufferung.

Die Makromoleküle können vorteilhafterweise natürliche oder synthetische Polyelektrolyte oder Polyampholyte umfassen, die stark oder schwach sein können, insbesondere synthetisches Polyelektrolyt Orotan 1850 oder organisches Polyelektrolyt D-Glucuronsäure, und/oder die Makromoleküle weisen einen isoelektrischen Punkt auf, der bei pH-Wert der Grundlösung zu einer Ladung führt, welche der Ladung zu trennender Partikel des biologischen Materials entspricht und/oder die Makromoleküle weisen ein Molekulargewicht von 10.000 bis 100.000 kDa, insbesondere von 30.000 bis 70.000 kDa auf. Es hat sich herausgestellt, dass solche Pufferlösungen zu besonders guten Ergebnissen führen.

Die Makromoleküle können ferner bevorzugt globuläre Proteine, insbesondere Albumine, bovines oder humanes Serumalbumin, Ovalbumin, Lactalbumin oder pflanzliche Albumine, β-Lactoglobulin, κ-Casein, Histone, Protamine, Globuline, Prolamine oder Gluteline mit einer Konzentration von 3 bis 7 Gew.-%, insbesondere 4 bis 5 Gew.-% umfassen. Alternativ oder zusätzlich umfassen die Makromoleküle noch bevorzugter filamentöse Proteine, insbesondere Gelatinen, Rindergelatinen, Schweinegelatinen oder Teleosteangelatinen in einer Konzentration von 0,3 bis 1,5 Gew.-%, insbesondere von 0,4 bis 0,8 Gew.-%, die eine niedrige Gelierkraft von 150 Bloom oder weniger, insbesondere 75 Bloom oder weniger aufweisen. Auch enzymatisch hydrolysiertes Kollagen (Kollagen-Hydrolysat) kann alternativ oder zusätzlich eingesetzt werden, vorzugsweise in einer Konzentration von 0,3 bis 20 Gew.-%, insbesondere von 1 bis 10 Gew.-%.

Makromoleküle dieser Arten, besonders in den angegebenen Konzentrationen und mit den daraus resultierenden Viskositäten, sind ganz besonders gut geeignet, die unspezifischen Bindungsstellen abzusättigen, und dabei mit einer passenden Fließgeschwindigkeit und Dichte Bedingungen zu erzeugen, in denen die Zielpartikel besonders gut an der Matrix anhaften können.

Bei dem erfindungsgemäßen Verfahren kann als Pufferlösung jede in der Beschreibung als geeignet genannte und insbesondere jede in Unteransprüchen zu der Vorrichtung beschriebene Pufferlösung verwendet werden.

Die Matrix und eine sie umgebende Trennsäule wird dabei bevorzugt vor dem Durchströmen der Suspension durch eine Vorinkubation mit reiner, also kein biologisches Material enthaltender Pufferlösung equilibriert, und zwar über einen ausreichend langen Zeitraum, um unspezifische Bindungsstellen in der Matrix abzusättigen, insbesondere über eine Dauer von 3 bis 20 oder 5 bis 10 Minuten, wobei die Matrix während des Equilibrierens ständig von Pufferlösung bedeckt gehalten wird. Die Suspension hat hier noch bevorzugter eine für den Trennvorgang kritische Konzentration des biologischen Materials, was auch von der verwandten Pufferlösung abhängig ist. Die angegebenen Zeitspannen genügen nach Erkenntnissen der Erfinder, um die spezifischen Bindungsstellen vorab hinreichend abzusättigen. Die vorbereitende Equilibrierung verhindert, dass sich anfänglich bei Einleiten der Suspension in die Matrix noch unerwünschte Partikel an den dann noch nicht vollständig von der Pufferlösung der Suspension abgesättigten unspezifischen Bindungsstellen anheften. Während des gesamten Verfahrens ist die Matrix bedeckt zu halten, damit die unspezifischen Bindungsstellen nicht freigegeben und die Flussbedingungen konstant gehalten werden, denn damit würde das Trennergebnis beeinträchtigt.

Zum Trennen von unerwünschtem biologischen Material wird das Eluat, also ein die Matrix verlassendes Fluid, bevorzugt nach dem Durchströmen aufgefangen, wobei nach Einführen der Suspension in die Matrix bei weiterhin aktiviertem Magnetfeld so lange die Matrix mit zusätzlicher reiner Pufferlösung durchströmt wird, bis sichergestellt ist, dass die Suspension die Matrix vollständig verlassen hat, und wobei die Matrix während des Durchströmens ständig von Pufferlösung bedeckt gehalten wird. Zielpartikel sind in diesem Fall Störkörper, die entfernt werden sollen, und sie haften in der Matrix, bis die eigentlich erwünschten Bestandteile des biologischen Materials vollständig aus der Matrix ausgespült sind. Das aufgefangene Eluat enthält die an der Matrix durch Magnetkraft haften gebliebenen Zielpartikel nicht mehr, sie werden abgetrennt, und ihre Konzentration ist im Eluat erheblich verringert, beziehungsweise das Eluat enthält im Idealfall gar keine solchen Zielpartikel mehr. Die in der Matrix verbliebenen Zielpartikel können anschließend getrennt entsorgt oder für alternative weitere Zwecke verwendet werden, indem sie in einem eigenen Arbeitsschritt ausgespült werden.

Alternativ wird zum Aufreinigen erwünschten biologischen Materials nach Einführen der Suspension in die Matrix bei noch aktiviertem äußeren Magnetfeld die Matrix so lange mit zusätzlicher reiner Pufferlösung durchströmt, bis sichergestellt ist, dass die Suspension die Matrix vollständig verlassen hat, und anschließend wird bei durch räumliche Trennung oder Abschalten deaktiviertem äußeren Magnetfeld die Matrix mit zusätzlicher reiner Pufferlösung ausgespült und dabei das Eluat aufgefangen, wobei die Matrix während des gesamten Vorgangs ständig von Pufferlösung bedeckt gehalten wird. In diesem Fall enthält das in der Phase des aktivierten Magnetfeld die Matrix verlassende Fluid kein primär interessierendes biologische Material, diese Fluid kann verworfen oder anderweitig verwendet werden. Erst beim anschließenden Ausspülen ohne Magnetfeld gibt die Matrix die Zielpartikel wieder frei, die also in dem dann aufgefangenen Eluat beim abschließenden Ausspülen der Matrix in einem ganz wesentlich erhöhten Reinheitsgrad enthalten sind. Die Pufferlösung bei diesem Ausspülen kann dieselbe, aber auch eine andere Pufferlösung sein wie während der eigentlichen HGMS-Phase mit aktiviertem Magnetfeld. Die Zielpartikel sind in diesem Fall in der anderen Pufferlösung des Eluats enthalten, oder in einer Mischung aus beiden Pufferlösungen.

Das aufgefangene Eluat wird bevorzugt zentrifugiert, und mit dem abzentrifugierten biologischen Material ohne Flüssigkeitsphase des Eluates wird das Verfahren bevorzugt ein- oder mehrfach wiederholt. Bei dem Trennverfahren verbleibt dann das biologische Material ohne die störenden, abzutrennenden Zielpartikel, dem Aufreinigungsverfahren verbleiben gerade umgekehrt die Zielpartikel in reiner Form ohne die Pufferlösung der Suspension.

Sofern der gewöhnlich schon nach einem Durchlauf hohe Trennungs- oder Aufreinigungsgrad noch nicht ausreicht, kann das Verfahren erneut ausgeführt werden. Dies gilt besonders dann, wenn die Matrix bei einem Trennverfahren überladen war, ansonsten sind nur noch deutlich verminderte Verbesserungen gegenüber dem ersten Durchlauf zu erwarten.

Das erfindungsgemäße Verfahren kann weiterhin mit ähnliche Merkmalen weitergebildet werden, wie sie beispielhaft, aber nicht abschließend in den sich an die unabhängigen Ansprüche anschließenden Unteransprüchen angegeben, und zeigt dabei ähnliche Vorteile.

Die Erfindung wird nachstehend auch hinsichtlich weiterer Merkmale und Vorteile anhand von Ausführungsbeispielen und unter Bezugnahme auf die Zeichnung beschrieben. Die Figuren der Zeichnung zeigen in:
- Fig. 1: eine schematische Frontalansicht des Aufbaus einer Ausführungsform der erfindungsgemäßen HGMS-Vorrichtung und der Trennsäule;
- Fig. 2A/B: eine flowzytometrische Analyse eines ersten Beispiels der erfindungsgemäßen Aufreinigung bei Verwendung einer isoosmolaren phosphatgepufferten Sucroselösung mit 0,75% Gelatine als Pufferlösung (Fig. 2A: *P. falciparum* Kultur vor Passage durch die HGMS-Säule ; Fig. 2B: Eluat nach Passage, Spülen und Entnahme der Trennsäule aus dem Magnetfeld),
- Fig. 3A/B: eine Darstellung gemäß Fig. 2 eines zweiten Beispiels mit bovinem Serumalbumin (BSA) enthaltender phosphatgepufferter Kochsalzlösung (PBS) als Pufferlösung;
- Fig. 4A/B: eine flowzytometrische Analyse eines dritten Beispiels als Dotplot (Fig. 4A: Anteil weißer CD8 positiver Blutzellen in einer Suspension von peripheren mononuklearen Zellen (PBMCs) vor Aufreinigung; Fig. 4B: Eluat aus der Trennsäule nach Passage der PBMCs, Spülen und Entnahme der Trennsäule aus dem Magnetfeld); Verwendung einer isoosmolaren phosphatgepufferten Sucroselösung mit 0,75% Gelatine als Pufferlösung wie im ersten Beispiel; und
- Fig. 5A/B: eine Darstellung gemäß Fig. 4 eines vierten Beispiels, jedoch Verwendung bovines Serumalbumin (BSA) enthaltender phosphatgepufferter Kochsalzlösung (PBS) als Pufferlösung wie im zweiten Beispiel.

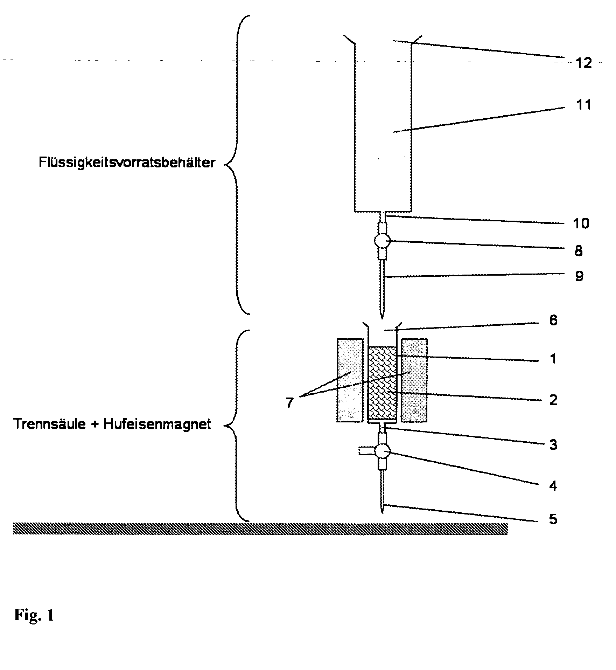

Figur 1 zeigt eine schematische Frontalansicht einer Ausführungsform der erfindungsgemäßen Aufreinigungsvorrichtung. Die Aufreinigungsvorrichtung umfasst eine Trennsäule 1, einen Flüssigkeitsvorratsbehälter 11, aus welchem Pufferlösung der Trennsäule 1 zugeführt werden kann, einen Permanent- oder Elektromagneten 7 zur Erzeugung eines starken magnetischen Feldes, sowie eine spezielle Pufferlösung, die weiter unten näher erläutert wird.

Die Trennsäule 1 weist ein Gehäuse und eine darin angeordnete Matrix 2 auf. Das Gehäuse besteht aus nichtmagnetischem Material und besitzt mindestens einen Flüssigkeitseinlass 6 und mindestens einen Flüssigkeitsauslass 3. Der Flüssigkeitsauslass 3 ist mit einer Vorrichtung 4 zur Beeinflussung der Fließgeschwindigkeit der Pufferlösung in der Trennsäule 1 versehen, welche einen Mehrwegehahn 4 und einem Durchflussbegrenzer 5 aufweist. Alternativ können auch andere bekannte Einrichtungen zur Regulierung der Fließgeschwindigkeit in der Trennsäule eingesetzt werden.

Die Matrix 2 ist so ausgebildet, das im Inneren der Trennsäule 1 ein hoher magnetischer Gradient von dem außen angeordneten Magnet erzeugt wird, wie dies zur HGM-Separation erforderlich ist. Dazu weist die Matrix 2 ferromagnetisches Material auf, etwa nichtrostenden, magnetischen Edelstahl oder anderes, welches geordnet oder ungeordnet, filamentös, sphärisch oder anderweitig geformt ist. Beispiele für die Ausführung der Matrix 2 werden weiter unten im Zusammenhang mit experimentellen Aufreinigungen genannt.

Die Trennsäule 1 mit der in ihr enthaltenen Matrix 2 befindet sich in einem starken, vorzugsweise homogenen magnetischen Feld, welches durch den Permanentmagneten oder Elektromagneten 7 erzeugt werden kann. Das Magnetfeld ist an- oder abschaltbar, beispielsweise durch Entfernung der Trennsäule aus dem Feld des Permanentmagneten, oder durch Abschalten des Elektromagneten 7.

Der Flüssigkeitsvorratsbehälter 11 weist einen Flüssigkeitseinlassbereich 12, einem Reservoirbereich und einem Flüssigkeitsauslass 10 auf, aus welchem die Pufferlösung in den Flüssigkeitseinlass 6 der Trennsäule 1 eingebracht werden kann. Der Flüssigkeitsauslass 10 des Flüssigkeitsvorratsbehälters 11 ist mit einer Einrichtung zur Beeinflussung der Ausflussgeschwindigkeit der in ihm enthaltenen Flüssigkeit versehen, welche einen Einwegehahn 8 und einen Durchflussbegrenzer 9 aufweist. Wie bei dem Flüssigkeitsauslass 3 der Trennsäule 1 sind auch hier andere bekannte Einrichtungen zur Regulierung der Ausflussgeschwindigkeit aus dem Flüssigkeitsvorratsbehälter 11 denkbar. Auch können jeweils nach Bedarf Ein- oder Mehrwegehähne verwendet bzw. gegeneinander ausgetauscht werden.

Die oben genannte erfindungsgemäß eingesetzte Pufferlösung dient zur Equilibrierung der Trennsäule 1 und Suspension des zu trennenden biologischen Materials. Diese Pufferlösung kann reines Wasser sowie eine oder mehrere verschiedene Sorten von Makromolekülen enthalten, welche in ausreichender Konzentration die Eigenschaft besitzen, unspezifische Bindungsstellen in der Trennsäule 1 und der darin befindlichen Matrix 2 absättigen zu können. Die Pufferlösung besitzt bevorzugt weiterhin eine Dichte, die der Dichte der zu trennenden Partikel hinreichend ähnlich ist, um die Auswirkungen der Schwerkraft auf die Partikel stark oder sogar möglichst vollständig zu reduzieren und daher selbige in Schwebe zu halten. Auf diese Weise wird eine Sedimentation der zu trennenden Partikel bei langsamen Fließgeschwindigkeiten in der Trennsäule 1 vermieden. Des weiteren besitzt die Pufferlösung bevorzugt eine Viskosität, welche zu einer für den Trennvorgang geeigneten Flussgeschwindigkeit mit laminaren Strömungsverhältnissen in der Trennsäule 1 führt. Schließlich können in der Pufferlösung verschiedene Ionen enthalten sein, wie beispielsweise, jedoch nicht ausschließlich, Kationen wie Natrium, Kalium, Magnesium und Calcium, oder Anionen wie Chlorid, Phosphat, Sulfat und Carbonat. Dabei soll vorgenannte Aufzählung keineswegs weitere andere Kationen oder Anionen ausschließen.

Die in der Pufferlösung zur Anwendung kommenden Makromoleküle sollten ausreichend groß sein, um unspezifische Bindungsstellen effektiv absättigen zu können, jedoch nicht so groß, dass sie in den zu verwendenden Konzentrationen die Viskosität der Pufferlösung zu kritisch erhöhen. Bevorzugt werden daher Makromoleküle mit einem Molekulargewicht von 10.000 bis 100.000 kDa, noch mehr bevorzugt mit einem Molekulargewicht von 30.000 bis 70.000 kDa. Vorteilhafterweise tragen die zugesetzten Makromoleküle eine Ladung, welche der Ladung der zu trennenden Partikel entspricht, da es sich vorzugsweise um eine kompetitive Hemmung der unspezifischen Bindung von zu trennenden Partikeln und Makromolekülen an das Material der Matrix 2 handelt. Beispielsweise werden, wenn es sich bei den zu trennenden Partikeln um Zellen handelt, deren Zellwand zumeist negative Ladungen trägt, vorzugsweise Makromoleküle mit negativer Ladung gewählt.

Solche positiv oder negativ geladenen Makromoleküle sind unter dem Oberbegriff Polyelektrolyte bekannt. Eine Untergruppe der Polyelektrolyte ist weiterhin unter dem Namen Polyampholyte bekannt. Dies bezeichnet Polyelektrolyte, die sowohl positive wie auch negative funktionelle Gruppen tragen. Die Nettoladung der Polyampholyte lässt sich bei Kenntnis ihres isoelektrischen Punktes und des pH-Werts der das Molekül umgebenden Pufferlösung leicht herleiten: Liegt der pH der Pufferlösung unterhalb des isoelektrischen Punktes des Polyampholyts, so liegt dessen Nettoladung im positiven Bereich, bei einem pH-Wert der Pufferlösung oberhalb des isoelektrischen Punktes verhält es sich umgekehrt, die Nettoladung liegt also im negativen Bereich. Liegt der pH-Wert der Pufferlösung am oder sehr in der Nähe des isoelektrischen Punktes des Polyampholyts, so ist dieser neutral, er trägt also keine Nettoladung.

Polyelektrolyte, welche sich im Rahmen der vorliegenden Erfindung insbesondere zur Auftrennung zellulären Materials vorteilhaft bewährt haben, sind beispielsweise Makromoleküle aus der Gruppe der Proteine (Eiweiße). Die Gruppe der Proteine wird in die beiden Untergruppen der globulären Proteine (kugelförmigen) und filamentösen Proteine (faserförmigen) unterteilt. Allerdings ist die begriffliche Trennung zwischen den genannten Untergruppen strenggenommen als unscharf zu anzusehen, da Übergangsformen zwischen kugelförmigen und faserförmigen Proteinen bestehen. Im Folgenden sollen daher die Begriffe "globulär" und "filamentös" Proteine der beiden Untergruppen sowie Übergangsformen mit umschließen.

In Flüssigkeiten gelöste globuläre und filamentöse Proteine besitzen nun die Eigenschaft, reversibel und irreversibel an feste Oberflächen zu binden, dieses Phänomen wird zumeist als Adsorption oder Adhäsion bezeichnet. Wissenschaftliche Veröffentlichungen weisen im Zusammenhang mit der Bindung an Edelstahloberflächen darauf hin, daß es bei Kontakt von in Flüssigkeiten gelösten Proteinen mit Edelstahloberflächen zur Abscheidung eines Monolayers (einer einschichtigen Molekülschicht) von Proteinmolekülen auf der Edelstahloberfläche kommt (Nakanishi et al., Journal of Bioscience and Bioengineering, 91, 2001:233-244, Fukuzaki et al., Journal of Fermentation and Bioengineering, 80, 1995:6-11). Während die Dicke dieses Monolayers im Bereich des hydrodynamischen Durchmessers (Stokes-Radius) des jeweiligen Proteinmoleküles liegt, sich also im Bereich weniger Nanometer bewegt, ist die genaue flächige Symmetrie des Monolayers bisher nicht genau bekannt. Es darf allerdings angenommen werden, dass der Monolayer nicht ganz regelmäßig ausgebildet wird. Pradier et al. weisen darauf hin, dass sogar möglicherweise Lücken zwischen den abgeschiedenen Molekülen bestehen, also die Edelstahloberfläche nicht zur Gänze mit Proteinmolekülen bedeckt sein könnte (Surface and Interface Analysis, 34, 2002:50-54). Weitere Untersuchungen lassen unklar, ob die Ausbildung eines solchen Monolayers die Korrosion der Edelstahloberfläche positiv oder negativ beeinflusst (Omanovic und Roscoe, Langmuir, 15, 1999:8315-8321, Hansen et al., Corrosion Science, 37, 1995:1423-1441). Insgesamt muss so davon ausgegangen werden, dass Wassermoleküle und gelöste Ionen keineswegs komplett von dem Monolayer ausgeschlossen werden. Trotzdem wurde im Rahmen der vorliegenden Erfindung festgestellt, dass die Ausbildung eines solchen Monolayers unspezifische Bindungen von nicht-Zielpartikeln an die Matrix eines HGMS-Separators äußerst effektiv zu verhindern vermag, darüber hinaus wurde weder eine physikalische noch eine chemische Schädigung des zu trennenden biologischen Materials beobachtet.

Im Folgenden soll auf einige Proteine, die sich im Rahmen der vorliegenden Erfindung besonders bewährt haben, näher eingegangen werden, ohne die Erfindung damit auf diese besonders geeigneten Proteine zu beschränken.

Als Vertreter aus der Untergruppe der globulären Proteine sind an dieser Stelle besonders bevorzugt zu nennen die Albumine, wie beispielsweise bovines und humanes Serumalbumin. Aus der Gruppe der Albumine weiterhin verwendbar, jedoch weniger bevorzugt sind Serumalbumine jeglicher anderer Spezies, sowie weiterhin andere Albumine, wie beispielsweise, jedoch nicht ausschließlich, Ovalbumin, Lactalbumin oder pflanzliche Albumine.

Für bovines oder humanes Serumalbumin in phosphatgepufferter isoosmolarer (physiologischer) Kochsalzlösung wurden im Rahmen der vorliegenden Erfindung Konzentrationen von 3% bis 7% und noch mehr bevorzugt von 4% bis 5% für den Trennvorgang von zellulärem Material als optimal gefunden. Deutlich niedrigere Konzentrationen (0,1%-1%), wie sie üblicherweise in der Zellforschung physiologischen Puffern zugesetzt werden, führten im Rahmen der vorliegenden Erfindung nicht zu dem gewünschten Ergebnis. Dies erklärt sich einerseits daraus, dass solch relativ niedrige Albuminkonzentrationen unspezifische Bindungsstellen der in dieser Erfindung eingesetzten Trennsäule 1 mit unbeschichteter Matrix 2 nicht effizient abzusättigen vermögen. Zum anderen verleihen diese niedrigen Albuminkonzentrationen der Pufferlösung nicht die notwendige Dichte, welche die zu trennenden Partikel in Schwebe zu halten und ein schwerkraftbedingtes Absinken auch bei langsamen Fließgeschwindigkeiten zu verhindern vermag.

Aus der Untergruppe der filamentösen Proteine sind im Rahmen der vorliegenden Erfindung besonders bevorzugt zu nennen die Gelatinen. Aufgrund ihres günstigen isoelektrischen Punktes von etwa pH 4,5 bis pH 5,6 und ihrer daher im neutralen pH-Bereich negativen Ladung finden sich unter den Gelatinen für Auftrennungen zellulären Materials im physiologischen pH-Bereich besonders bevorzugt Gelatinen der Klasse B (Rindergelatinen). Ein Konzentrationsbereich, welcher besonders günstig für den Trennvorgang zellulären Materials ist, wurde für Rindergelatinen von 0,3% bis 1,5%, noch mehr bevorzugt von 0,4% bis 0,8% gefunden. Andere Gelatinen wie Gelatinen der Klasse A (Schweinegelatinen) oder Teleosteangelatinen (Fischgelatinen), oder jegliche andere Gelatinen sowie die enzymatisch hydrolysierten Kollagene (Kollagen-Hydrolysate) als eine weitere Untergruppe der Gelatinen sind gleichfalls einsetzbar, werden jedoch aufgrund ihrer weniger günstigen isoelektrischen Punkte zumindest für Auftrennungen im physiologischen pH-Bereich weniger bevorzugt. Generell sind unter den verschiedenen Gelatinen B sowie den Gelatinen der anderen genannten Gruppen jene zu bevorzugen, welche eine niedrige Gelierkraft (Bloomstärke) aufweisen, vorzugsweise weniger als 150 Bloom und noch weiter bevorzugt weniger als 75 Bloom.

Weitere Makromoleküle aus der Gruppe der Proteine, welche im Rahmen der vorliegenden Erfindung eingesetzt werden können, sind in nicht abschließender Aufzählung unter anderem weitere globuläre Proteine, wie beispielsweise β-Lactoglobulin oder κ-Casein, oder andere globuläre Proteine aus den Untergruppen der Histone und Protamine, der Globuline, der Prolamine und der Gluteline, sowie weitere filamentöse Proteine.

In weiteren Ausführungsformen der Erfindung ist der Einsatz anderer Makromoleküle aus der Gruppe der organischen oder synthetischen Polyelektrolyte denkbar, beispielsweise des synthetischen Polyelektrolyts Orotan 1850™ (Rohm and Haas, Philadelphia, PA, USA) oder des organischen Polyelektrolyts D-Glucuronsäure.

Einige der erwähnten Makromoleküle, die zur Verminderung der unspezifischen Bindung des zu trennenden Materials an das Matrixmaterial der Pufferlösung zugesetzt werden können, führen in Kombination mit bestimmten Pufferlösungen zu einer Aggregation des zu trennenden, sich in Suspension befindlichen biologischen Materials. Die Aggregationsneigung von roten Blutzellen in Suspension korreliert positiv mit dem Molekulargewicht und der Konzentration der zugesetzten Makromoleküle sowie mit der Ionenstärke der Pufferlösung, wobei sich die Ionenstärke dabei als die Gesamtladungskonzentration der gelösten Ionen in der Pufferlösung versteht. Niedrige Ionenstärken können einer Aggregation, welche durch zugesetzte Makromoleküle verursacht wird, entgegenwirken und vermögen diese sogar gänzlich zu verhindern. Die Betrachtung des hydrodynamischen Durchmessers (Stokes-Radius), welcher sich von dem Molekulargewicht und der Messung der intrinsischen Viskosität herleiten lässt, ermöglicht noch genauer als die alleinige Betrachtung des Molekulargewichts eines bestimmten Makromoleküls dessen aggregationsfördernde Wirkung auf eine Suspension von roten Blutzellen vorherzusagen (vgl. hierzu auch die Arbeiten von Jan und Chien (Journal of General Physiology, 61, 1973:638-654, 655-668 und Armstrong et al. (Biophysical Journal, 87, 2004:4259-4270)). Der Begriff "Molekulargewicht" eines Makromoleküls soll im Folgenden immer auch stellvertretend für den daraus ableitbaren hydrodynamischen Durchmesser (Stokes-Radius) dieses Makromoleküls stehen.

Es hat sich im Rahmen der Erfindung als vorteilhaft herausgestellt, diese Erkenntnisse auszunutzen und die Ionenstärke der Pufferlösung anzupassen, um einer Aggregation des zu trennenden biologischen Materials wirksam vorzubeugen. Beispielsweise haben die Erfinder eine Aggregation von Blutzellen insbesondere bei Suspension in gelatinehaltiger physiologischer phosphatgepufferter Kochsalzlösung beobachtet. Um diese Aggregation zu verhindern, erwies es sich zur Auftrennung von Blutzellen unter der Verwendung von Gelatinen als besonders vorteilhaft, physiologische phosphatgepufferte Kochsalzlösung durch physiologische phosphatgepufferte Sucroselösung ganz- oder teilweise zu ersetzen.

Aus dem beschriebenen Zusammenhang zwischen Partikelaggregation und den speziellen Eigenschaften der Pufferlösung (insbesondere der Konzentration und dem Molekulargewicht der Makromoleküle, sowie der Ionenstärke der Pufferlösung) können Vorhersagen getroffen werden, welche Kombination von Makromolekülen und Pufferlösungen für die Auftrennung eines bestimmten zu trennenden biologischen Materials besonders geeignet sind, und es kann eine makromolekülhaltige Pufferlösung gewählt werden, deren physikalisch-chemische Eigenschaften an die für ein bestimmtes Partikel benötigten Trennbedingungen angepasst sind. Der Begriff physikalisch-chemische Eigenschaften einer Pufferlösung soll dabei im Rahmen der vorliegenden Erfindung Viskosität, Dichte, Ionenstärke, Osmolarität und pH-Wert der Flüssigkeit, weiterhin Art, Molekulargewicht, Ladung, und Konzentration der in dieser gelösten Makromoleküle und andere beeinflussbare physikalisch-chemische Parameter sowohl der Makromoleküle als auch der diese umgebenden Flüssigkeit mit umschließen.

Im Folgenden wird das erfindungsgemäße Aufreinigungsverfahren schrittweise näher erläutert.

In einem ersten Schritt wird die Trennsäule 1 mit Pufferlösung zur Absättigung unspezifischer Bindungsstellen equilibriert. Die Equilibrierung erfolgt dabei durch eine Vorinkubation der Trennsäule 1 mit einer wie oben beschriebenen, für den Trennvorgang der spezifischen zu untersuchenden Partikelsuspension geeigneten Pufferlösung, im Folgenden genannt Pufferlösung A, über einen ausreichend langen Zeitraum, welcher sich nach den verwandten Makromolekülen in der Pufferlösung richtet. Im Regelfall beträgt dieser Zeitraum 3 bis 20 Minuten, davon noch regelmäßiger 5 bis 10 Minuten, in seltenen Fällen können jedoch auch kürzere oder längere Zeiträume notwendig sein.

Nach der Equilibrierung wird das zu trennende biologische Material in Pufferlösung A suspendiert und in den Einlassbereich 6 der Trennsäule 1 gegeben, wobei sich die Trennsäule 1 im homogenen Magnetfeld des Hufeisen- oder Elektromagneten 7 befindet. Gleichzeitig wird der Flüssigkeitsauslass 3 der Trennsäule 1 mit Hilfe des Mehrwegehahns 4 geöffnet. Dabei ist darauf zu achten, die Matrix 2 ständig von Pufferlösung bedeckt zu halten. Das biologische Material fließt durch die Trennsäule 1, wobei sich die intrinsisch paramagnetischen oder vorab mit synthetischen paramagnetischen Teilchen markierten Zielpartikel an die Matrix 2 anheften. Alle unmagnetischen Partikel durchlaufen die Trennsäule 1 jedoch ungehindert und treten am Flüssigkeitsauslass 3 aus.

Nach vollständiger Zugabe des zu trennenden biologischen Materials erfolgt das Spülen der Trennsäule 1. Dieses dient dem Ausspülen der noch in der Trennsäule 1 verbliebenen unmagnetischen nicht-Zielpartikel. Der Flüssigkeitsauslass 10 des Flüssigkeitsvorratsbehälters 11, enthaltend Pufferlösung A, wird mit Hilfe des Einwegehahns 8 geöffnet, so dass nun reine Pufferlösung A als Spüllösung durch die Trennsäule 1 fließt. Dabei ist wieder darauf zu achten, die Matrix 2 ständig von Pufferlösung bedeckt zu halten. Die Menge der zur Spülung zu verwendenden Pufferlösung A richtet sich nach der Größe der Trennsäule 1. Beispielsweise beträgt die zu einer ausreichenden Spülung notwendige Menge an Pufferlösung A für eine Trennsäule 1 mit einem Volumen von 3 ml etwa 30-60 ml, für Trennsäulen anderer Größe muss sie entsprechend angepasst werden.

Nach Abschluss des Spülens der Trennsäule 1 wird der Flüssigkeitsauslass 3 der Trennsäule 1 und des Flüssigkeitsvorratsbehälters 11 geschlossen. Die Trennsäule 1 wird aus dem Magnetfeld des Hufeisenmagneten 7 entnommen, oder im Falle der Verwendung eines Elektromagneten 7 das Magnetfeld abgeschaltet. Ohne den Einfluss des Magnetfeldes lösen sich nun die Zielpartikel von der Matrix 2 und können durch antero- oder retrogrades Spülen der Trennsäule 1 mit Pufferlösung A oder, da die Trennung nun vollzogen ist, einer beliebigen anderen Pufferlösung ausgewaschen werden.

Dieses Verfahren eignet sich sowohl zur Aufreinigung von Zielpartikeln, da diese im Eluat enthalten sind, welches durch das Auswaschen der Säule erhalten wurde, als auch zur Entfernung von Zielpartikeln aus dem zu trennenden biologischen Material. Im zweiten Falle ist die aus der Trennsäule 1 austretende Spüllösung von Interesse, die das um die Zielpartikel reduzierte biologische Material enthält. Die Spüllösung wird dann während des Einbringens des in Pufferlösung A suspendierten biologischen Materials in die Trennsäule 1 und während des anschließenden Spülens der Trennsäule 1 mit reiner Pufferlösung A aufgefangen. Dabei ist wichtig zu beachten, dass die Matrix 2 nicht mit einer zu hohen Zahl von Zielpartikeln überladen wird, da bei Erschöpfung der Kapazität der Matrix 2 nicht gebundene Zielpartikel mit dem übrigen zu trennenden biologischen Material aus dem Flüssigkeitsauslass 3 der Trennsäule 1 austreten können.

Das Verfahren kann mit den gewonnenen, aufgereinigten Zielpartikeln wiederholt werden, falls eine noch höhere Aufreinigung erzielt werden soll. Gleiches gilt für die aufgefangene Spüllösung nach Durchlaufen durch die Trennsäule 1, falls eine noch reinere Entfernung von Zielpartikeln aus dem zu trennenden biologischen Material angestrebt wird. Das ist aber wegen des hohen erfindungsgemäßen Aufreinigungsgrads oft nicht mehr erforderlich.

Die nachfolgenden Versuchsbeispiele sollen die Erfindung weiter illustrieren, ohne aber damit die Anwendbarkeit auf die Versuchsbeispiele einzuschränken.

### Beispiel 1

Aufreinigung von mit Malariaerregern (Plasmodien) infizierten roten Blutzellen aus einer *P*. *falciparum* Kultur mit isoosmolarer gelatinehaltiger phosphatgepufferter Sucroselösung.

### Material:

Pufferlösung A₁: isoosmolare phosphatgepufferte Sucroselösung mit 0,75% Gelatine
Edelstahlwolle 1 g
Einwegehahn
Dreiwegehahn
20G Injektionsnadel
3 ml Einwegspritze
10 ml Einwegspritze
50 ml Einwegspritze
1 Neodymium-Hufeisenmagnet

### a) Bereitstellung des Aufreinigungskits

### Herstellung der Trennsäule

Eine 3 ml Einwegspritze als Trennsäule 1 wurde zu zwei Dritteln ihres Gesamtvolumens mit einem Gramm Edelstahlwolle als Matrix 2 gefüllt. Dabei wurde darauf geachtet, dass die Mehrzahl der Edelstahlwollefasern in Längsrichtung des Spritzenkörpers zu liegen kommt. An den Flüssigkeitsauslass 3 des Spritzenkörpers wurde ein Dreiwegehahn 4 und die 20G Injektionsnadel als Durchflussbegrenzer 5 angeschlossen. Das obere, nicht von Edelstahlwolle gefüllte Drittel der Einwegspritze diente als Einlassbereich 6 der Trennsäule 1.

### Equilibrierung der Trennsäule

Über den Dreiwegehahn 4 wurde die so hergestellte Trennsäule 1 mit Pufferlösung in senkrechter Position retrograd gefüllt. Luftblasen wurden durch Klopfen mit dem Finger möglichst vollständig evakuiert, wobei allerdings gefunden wurde, dass verbleibende kleinere Luftblasen den Trennvorgang nicht negativ beeinflussen. Die Trennsäule 1 wurde nun zwischen den Polen des Hufeisenmagneten 7 positioniert, so dass die gesamte Matrix 2 dem homogenen Magnetfeld ausgesetzt war, und 10 Minuten equilibriert.

### Aufbau und Positionierung des Flüssigkeitsvorratsbehälters

Ein Einwegehahn 8 versehen mit einer 18G Nadel als Durchflussbegrenzer 9 wurde am Flüssigkeitsauslass 10 der 50 ml Einwegspritze, welche als Flüssigkeitsvorratsbehälter 11 diente, angebracht. Diese wurde dann so über der Trennsäule 1 positioniert, dass die aus der Nadel austretende Flüssigkeit in den Einlassbereich 6 der Trennsäule 1 tropfen konnte.

### b) Vorbereitung und Durchführung des Trennvorganges

### Vorbereitung der P. falciparum Kultur zur Aufreinigung von mit Malariaerregern infizierten roten Blutzellen

50 µl rote Blutzellen einer *P. falciparum* Kultur mit einer Parasitämie von 13,51% wurden in 450 µl RPMI Medium resuspendiert und 10 min unter Einfluss von Raumluft oxygeniert. Die Kultur wurde sodann abzentrifugiert und in 5 ml Pufferlösung A₁ resuspendiert.

### Durchführung des Trennvorganges

Nach Abschluss der 10minütigen Equilibrierung der Trennsäule 1 wurden deren Flüssigkeitsauslass 3 geöffnet und gleichzeitig die resuspendierten Zellen langsam so in den Einlassbereich der Trennsäule 1 eingebracht, dass die Matrix 2 ständig von Flüssigkeit bedeckt blieb. Nach erfolgter vollständiger Zugabe der resuspendierten Kultur wurde der Flüssigkeitsauslass 10 des Flüssigkeitsvorratsbehälters 11, welcher 45 ml Pufferlösung A₁ enthielt, geöffnet. Dabei war zu vermeiden, die Fließgeschwindigkeit in der Trennsäule 1 durch Manipulation am Flüssigkeitsauslass 3 der Trennsäule 1 zu ändern, und darauf zu achten, die Matrix 2 der Trennsäule 1 ständig von Pufferlösung A₁ bedeckt zu halten. Nach vollständiger Zugabe der Pufferlösung A₁ aus dem Flüssigkeitsvorratsbehälter 11 wurde der Fluss in der Trennsäule 1 durch Schließen ihres Flüssigkeitsauslasses 3 gestoppt und die Trennsäule 1 aus dem Magnetfeld entnommen. Eine 10 ml Einwegspritze, welche mit Pufferlösung A₁ gefüllt ist, wurde am Dreiwegehahn 4 angesetzt und die Trennsäule retrograd 1 ausgespült. Das Eluat wurde dabei in einem geeigneten Gefäß aufgefangen und die Suspension 5 min bei 1500 g abzentrifugiert. Der Überstand wurde verworfen und das Pellet in 300 µl phosphatgepufferter Kochsalzlösung resuspendiert.

### c) Auswertung und Ergebnis

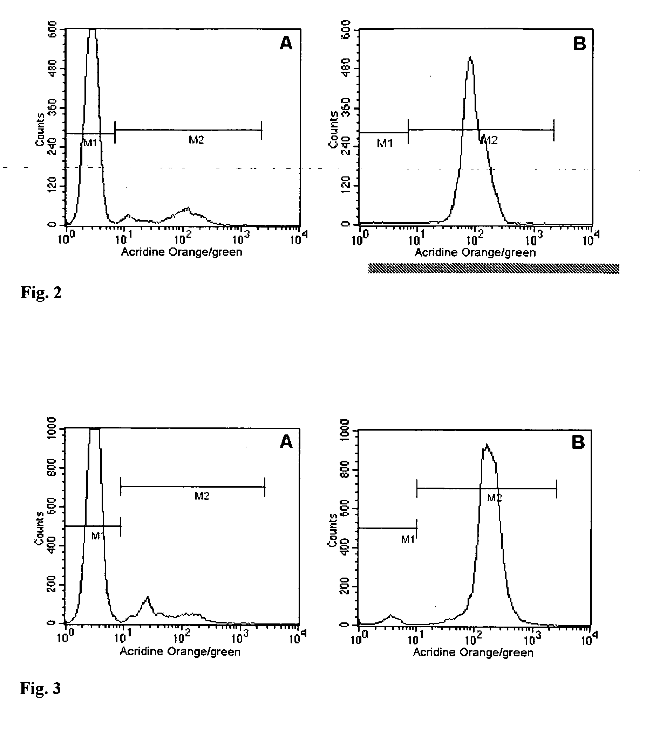

Es erfolgte ein Anfärben der Zellen mit Acridine Orange und eine flowzytometrische Analyse wie anderweitig beschrieben (Bhakdi et al., Cytometry A 2007: (71A) 662-667). Fig. 2 zeigt das Ergebnis der Aufreinigung anhand von Histogrammen der flowzytometischen Analyse. A) *P. falciparum* Kultur vor Passage durch die Hochgradientenmagnetseparationssäule. M1: normale rote Blutzellen, M2: mit *P. falciparum* infizierte rote Blutzellen (13,51%). B) Eluat aus der Trennsäule 1 nach Passage der Kultur, Spülen mit 45 ml Pufferlösung A₁ und Entnahme der Trennsäule 1 aus dem Magnetfeld. M1: normale rote Blutzellen, M2: Mit *P. falciparum* infizierte rote Blutzellen, aufgereinigt auf 99,54%. Entsprechend zeigte ein mit Giemsa gefärbter Blutausstrich ausschließlich mit Malariaerregern infizierte rote Blutzellen. Die Malariaerreger in den aufgereinigten roten Blutzellen ließen sich über mehrere Tage problemlos weiter kultivieren.

### Beispiel 2

Aufreinigung von mit Malariaerregern infizierten roten Blutzellen aus einer *P. falciparum* Kultur mit bovines Serumalbumin (BSA) enthaltender phosphatgepufferter Kochsalzlösung (PBS).

### Material:

Wie aufgelistet in Beispiel 1, jedoch wurde Pufferlösung A₁ mit Pufferlösung A₂ (PBS mit 5% BSA) ersetzt.

### a) Bereitstellung des Aufreinigungskits

Wie beschrieben in Beispiel 1

### b) Vorbereitung und Durchführung des Trennvorganges

### Vorbereitung der P. falciparum Kultur zur Aufreinigung von mit Malariaerregern infizierten roten Blutzellen

Wie beschrieben in Beispiel 1. Die Parasitämie der *P. falciparum* Kultur betrug in diesem Versuch 14.47%. Pufferlösung A₁ wurde durch Pufferlösung A₂ ersetzt.

### Durchführung des Trennvorganges

Wie beschrieben in Beispiel 1, mit folgenden Unterschieden:
Pufferlösung A₁ wurde durch Pufferlösung A₂ ersetzt. Das Abzentrifugieren des Eluates erfolgte dann bei 800 g für 5 min.

### c) Auswertung und Ergebnis

Die Auswertung erfolgte analog zu Beispiel 1. Fig. 3 zeigt das Ergebnis der Aufreinigung anhand von Histogrammen der flowzytometischen Analyse. A) *P. falciparum* Kultur vor Passage durch die Hochgradientenmagnetseparationssäule. M1: normale rote Blutzellen, M2: mit *P. falciparum* infizierte rote Blutzellen (14,47%). B) Eluat aus der Trennsäule 1 nach Passage der Kultur, Spülen mit 45 ml Pufferlösung A₂ und Entnahme der Trennsäule 1 aus dem Magnetfeld. M1: normale rote Blutzellen, M2: Mit *P. falciparum* infizierte rote Blutzellen, aufgereinigt auf 97,03%. Entsprechend zeigte ein mit Giemsa gefärbter Blutausstrich nahezu ausschließlich mit Malariaerregern infizierte rote Blutzellen. Die Malariaerreger in den aufgereinigten roten Blutzellen ließen sich über mehrere Tage problemlos weiter kultivieren.

### Beispiel 3

Aufreinigung von weißen Blutzellen, welche das Oberflächenantigen CD8 tragen (CD8 positive Zellen) aus einer Suspension von weißen Blutzellen (peripheren mononuklearen Blutzellen (PBMCs)).

### Material:

Wie aufgelistet in Beispiel 1

### a) Bereitstellung des Aufreinigungskits

Wie beschrieben in Beispiel 1.

### b) Vorbereitung und Durchführung des Trennvorgangs

### Markieren von CD8 positiven Zellen mit Antikörper-konjugierten synthetischen paramagnetischen Teilchen (Microbeads)

1,5x10⁷ humane periphere mononukleare Zellen (PBMCs) wurden mit monoklonalen Ratteanti-Mensch CD8 IgG Antikörpern 30 Minuten in PBS/BSA 1% auf Eis inkubiert. Die Zellen wurden zweimal mit der gleichen Pufferlösung gewaschen und anschließend mit anti-Ratte IgG konjugierten Microbeads (Miltenyi Biotech GmbH, loc. cit.) für weitere 10 Minuten auf Eis inkubiert. Die Zellen wurden erneut zweimal in gleicher Pufferlösung gewaschen und schließlich weitere 30 Minuten mit fluoreszenzmarkierten Antikörpern (PE-anti CD8 und FITC-anti CD3, Simultest^{™} BD Biosciences, 2350 Qume Drive, San Jose, CA USA 95131) auf Eis inkubiert. Es folgte erneut ein zweimaliges Waschen der Zellen mit PBSBSA 1%. Die Zellen wurden dann nochmals abzentrifugiert und in 1,5 ml Pufferlösung A₁ resuspendiert.

### Durchführung des Trennvorganges

Wie beschrieben in Beispiel 1

### c) Auswertung und Ergebnis

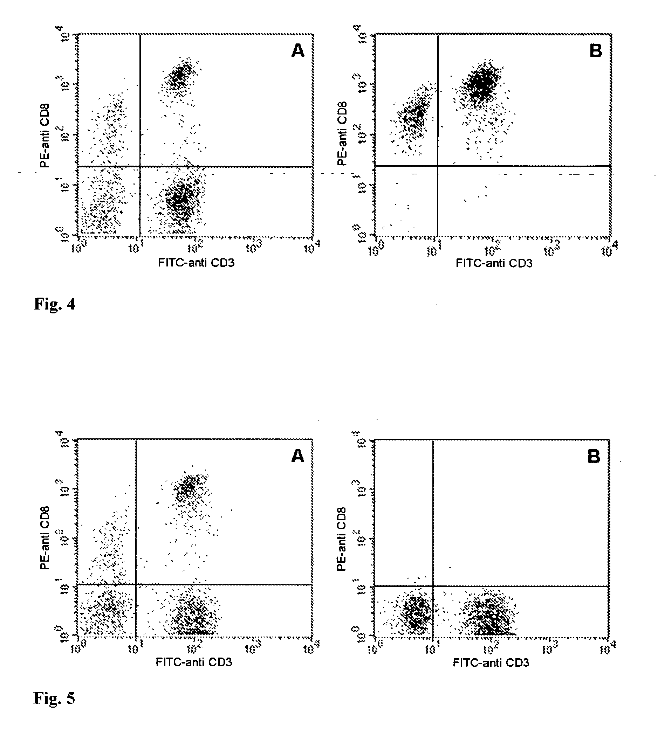

Fig. 4 zeigt das Ergebnis der Aufreinigung der CD8 positiven Zellen aus der Suspension von PBMCs. A) Zellpopulation der Probe. Die beiden oberen Quadranten zeigen die CD8 positiven Zellen (23,21%). B) Eluat aus der Trennsäule 1 nach Passage der PBMCs, Spülen mit 45 ml Pufferlösung A₁ und Entnahme der Trennsäule 1 aus dem Magnetfeld. Die beiden oberen Quadranten zeigen die CD8 positiven Zellen, aufgereinigt auf 99,17%.

### Beispiel 4

### Entfernung (Depletion) von CD8 positiven Zellen aus einer Suspension von PBMCs

### Material

Wie aufgelistet in Beispiel 2, jedoch Einsatz einer 25G Injektionsnadel als Durchflussbegrenzer anstelle einer 20G Injektionsnadel.

### A) Bereitstellung des Aufreinigungskits

Wie beschrieben in Beispiel 1.

### B) Vorbereitung und Durchführung des Trennvorganges

### Markieren von CD8 positiven Zellen mit Antikörper-konjugierten synthetischen paramagnetischen Teilchen (Microbeads)

Wie beschrieben in Beispiel 3.

### Durchführung des Trennvorganges

Wie beschrieben in Beispiel 2, jedoch Einsatz von 15 ml Pufferlösung A₂. Für diesen Versuch war die die Trennsäule 1 durchlaufende Flüssigkeit von Interesse. Sie wurde in einem geeigneten Gefäß aufgefangen, die Zellen bei 800 g für 5 min abzentrifugiert und in 300 µl PBS resuspendiert.

### c) Auswertung und Ergebnis

Fig. 5 zeigt das Ergebnis der Entfernung der CD8 positiven Zellen aus der Suspension von PBMCs. A) Zellpopulation der Probe. Die beiden oberen Quadranten zeigen die CD8 positiven Zellen (35,41%). B) Eluat aus der Trennsäule 1 nach Passage der PBMCs, Spülen mit 15 ml Pufferlösung A₂ und Entnahme der Trennsäule 1 aus dem Magnetfeld. Die beiden oberen Quadranten zeigen die CD8 positiven Zellen, depletiert auf 0,57%.

Alle vorangehend erwähnten Veröffentlichungen sind durch Verweis in Gänze in die vorliegende Patentschrift aufgenommen.

Zusammengefasst beruht die Erfindung auf dem Gedanken, eine technische Vorrichtung mit Verbrauchsmaterialien zur Durchführung einer Hochgradientenmagnetseparation an biologischem Material einzusetzen, auch bezeichnet als "Kit", umfassend oder bestehend aus:
1.1. einer Trennsäule enthaltend eine Matrix bestehend aus ferromagnetischem Material, welches zur Generierung eines Hochgradientenmagnetfeldes innerhalb eines externen starken, homogenen magnetischen Feldes geeignet ist,
1.2. einem Flüssigkeitsvorratsbehälter, aus welchem Pufferlösung A in den Einlassbereich der Trennsäule eingebracht werden kann,
1.3. einem Permanent- oder Elektromagneten zur Erzeugung eines starken, homogenen magnetischen Feldes,
1.4. einer Pufferlösung A zur Equilibrierung der Trennsäule und Suspension des zu trennenden biologischen Materials,
bei dem die Pufferlösung A mindestens eine der drei folgenden vorteilhaften Eigenschaften aufweist:
1.5. Pufferlösung A enthält zur Equilibrierung der Trennsäule und zur Suspension des zu trennenden biologischen Materials Makromoleküle, welche die Eigenschaft besitzen, unspezifische Bindungsstellen in der Trennsäule absättigen zu können, und/oder
1.6. Pufferlösung A besitzt eine Dichte, die der Dichte der zu trennenden Partikel hinreichend ähnlich ist, um die Auswirkungen der Schwerkraft auf die Partikel so weit wie möglich zu reduzieren und daher selbige in der Pufferlösung A in Schwebe zu halten, und/oder
1.7. besagte Pufferlösung A besitzt eine Viskosität, welche zu einer für den Trennvorgang geeigneten Flussgeschwindigkeit mit laminaren Strömungsverhältnissen in der Trennsäule beiträgt.

## Patentansprüche

1. Hochgradientenmagnetseparations-Vorrichtung für die Trennung oder Aufreinigung magnetischen oder magnetisch markierten biologischen Materials mit einem Magnet (7), einer Trennsäule (1) und einer in einem Innenraum der Trennsäule anordenbaren ferromagnetischen Matrix (2), sowie mit einem eine Pufferlösung zur Equilibrierung der Trennsäule (1) und/oder zur Suspension des biologischen Materials enthaltenden Vorratsbehälter (11), wobei im Betrieb ein von dem Magnet (7) erzeugtes Magnetfeld in der Matrix (2) ein Hochgradientenmagnetfeld erzeugen und Pufferlösung aus dem Vorratsbehälter (11) die Trennsäule (1) durchströmen kann,
**dadurch gekennzeichnet,**
**dass** die Pufferlösung eine Grundlösung und Makromoleküle umfasst, welche unspezifische Bindungsstellen der Matrix (2) absättigen können, wobei die Makromoleküle filamentöse Proteine umfassen, insbesondere Gelatine.

2. Vorrichtung nach Anspruch 1,
wobei die Pufferlösung eine Dichte aufweist, die der Dichte zu trennender Partikel des biologischen Materials so weitgehend entspricht, dass eine auf die Partikel wirkende Schwerkraft im Wesentlichen kompensiert ist und die Partikel daher in der Pufferlösung nahezu schweben, und/oder die Pufferlösung eine hohe Viskosität aufweist, welche ein laminares Durchströmen der Trennsäule (1) mit für den Trennvorgang geeigneter Fließgeschwindigkeit ermöglicht.

3. Vorrichtung nach Anspruch 1 oder 2,
wobei der Magnet (7) ein Permanent- oder Elektromagnet ist, welcher so geformt ist, dass die Trennsäule (1) in dem von ihm erzeugten insbesondere homogenen Magnetfeld angeordnet werden kann, wobei die Trennsäule (1) durch räumliche Trennung und/oder Abschalten wahlweise unter Einwirkung des Magnetfelds stehen kann oder nicht.

4. Vorrichtung nach einem der vorhergehenden Ansprüche,
wobei die Matrix (2) unbeschichtet ist und/oder geordnetes oder ungeordnetes, filamentöses, sphärisches oder anderweitig geformtes Material aufweist, insbesondere nichtrostenden, magnetischen Edelstahl oder Stahlwolle.

5. Vorrichtung nach einem der vorhergehenden Ansprüche,
wobei der Vorratsbehälter (11) mit der Trennsäule (I) verbunden ist, so dass Pufferlösung über eine Zuflussbegrenzungseinrichtung (8, 9) mit einer einstellbaren Fließgeschwindigkeit in die Trennsäule (1) eingebracht werden kann oder nicht, und/oder wobei die Trennsäule (1) eine Durchflussbegrenzungseimichtung (4, 5) aufweist, welche den Ausfluss aus der und die Strömungsgeschwindigkeit in der Trennsäule (1) beeinflussen kann.

6. Vorrichtung nach einem der vorhergehenden Ansprüche,
wobei die Ionenstärke der Grundlösung in Abhängigkeit von den Makromolekülen so angepasst ist, dass aggregierende Effekte der Makromoleküle auf das biologische Material kompensiert werden, wobei die Grundlösung eine isoosmolare Konzentration aus Kationen Natrium, Kalium, Magnesium oder Kalzium und aus Anionen Chlorid, Phosphat, Sulfat oder Carbonat aufweist, insbesondere eine phosphatgepufferte Kochsalz- oder Sucroselösung oder eine Mischung daraus ist.

7. Vorrichtung nach einem der vorhergehenden Ansprüche,
wobei die Makromoleküle natürliche oder synthetische Polyelektrolyte oder Polyampholyte umfassen, insbesondere synthetisches Polyelektrolyt oder organisches Polyelektrolyt D-Glucuronsäure, und/oder wobei die Makromoleküle einen isoelektrischen Punkt aufweisen, der bei pH-Wert der Grundlösung zu einer Ladung führt, welche der Ladung zu trennender Partikel des biologischen Materials entspricht und/oder wobei die Makromoleküle ein Molekulargewicht von etwa 10.000 bis etwa 100.000 kDa, insbesondere von etwa 30.000 bis etwa 70.000 kDa aufweisen.

8. Vorrichtung nach einem der vorhergehenden Ansprüche,
wobei die Makromoleküle globuläre Proteine umfassen, insbesondere Albumine, bovines oder humanes Serumalbumin, Ovalbumin, Lactalbumin oder pflanzliche Albumine, β-Lactoglobulin, κ-Casein, Histone, Protamine, Globuline, Prolamine oder Gluteline mit einer Konzentration von etwa 3% bis etwa 7%, insbesondere etwa 4% bis etwa 5% bezogen auf die Pufferlösung.

9. Vorrichtung nach einem der vorhergehenden Ansprüche,
wobei die filamentösen Proteine hydrolisierte Kollagene in einer Konzentration von etwa 0,3 bis etwa 20 Gew.-%, insbesondere von etwa 1 bis etwa 10 Gew.-%., umfassen.

10. Vorrichtung nach einem der vorhergehenden Ansprüche,
wobei die Gelatinen, Rindergelatinen, Schweinegelatinen oder Teleosteangelatinen und/oder eine Konzentration von etwa 0,3% bis etwa 1,5%, insbesondere von etwa 0,4% bis etwa 0,8%, und/oder eine niedrige Gelierkraft von etwa 150 Bloom oder weniger, insbesondere etwa 75 Bloom oder weniger aufweisen.

11. Verfahren zur Trennung oder Aufreinigung intrinsisch magnetischen oder vorbereitend magnetisch markierten biologischen Materials mittels Hochgradientenmagnetseparation, wobei eine Suspension mit dem biologischen Material eine in einem äußeren Magnetfeld angeordnete ferromagnetische Matrix (2) durchströmt, so dass das Material an der Matrix (2) anhaftet,
**dadurch gekennzeichnet,**
**dass** die Matrix (2) unbeschichtet ist, dass das biologische Material in einer Pufferlösung mit einer Grundlösung und Makromolekülen suspendiert wird, die beim Durchströmen der Matrix unspezifische Bindungsstellen der Matrix (2) absättigen und dass die Matrix (2) und eine sie umgebende Trennsäulen (1) vor dem Durchströmen der Suspension durch eine Vorinkubation mit reiner, also kein biologisches Material enthaltender Pufferlösung über einen ausreichend langen Zeitraum equilibriert wird, um unspezifische Bindungsstellen in der Matrix (2) abzusättigen.

12. Verfahren nach Anspruch 11,
wobei die Matrix (2)
über eine Dauer von etwa 3 bis etwa 20 oder etwa 5 bis etwa 10 Minuten equilibriert wird, und wobei die Matrix (2) während des Equilibrierens ständig von Pufferlösung bedeckt gehalten wird.

13. Verfahren nach Anspruch 11 oder 12,
wobei zum Trennen von unerwünschtem biologischen Material das Eluat, also ein die Matrix (2) verlassendes Fluid, nach dem Durchströmen aufgefangen wird, wobei nach Einrühren der Suspension in die Matrix (2) bei weiterhin aktiviertem Magnetfeld so lange die Matrix (2) mit zusätzlicher reiner Pufferlösung durchströmt wird, bis sichergestellt ist, dass die Suspension die Matrix (2) vollständig verlassen hat, und wobei die Matrix (2) während des Durchströmens ständig von Pufferlösung bedeckt gehalten wird.

14. Verfahren nach Anspruch 11 oder 12,
wobei zum Aufreinigen erwünschten biologischen Materials nach Einführen der Suspension in die Matrix. (2) bei noch aktiviertem äußeren Magnetfeld die Matrix (2) so lange mit zusätzlicher reiner Pufferlösung durchströmt wird, bis sichergestellt ist, dass die Suspension die Matrix (2) vollständig verlassen hat, und anschließend bei durch räumliche Trennung oder Abschalten deaktiviertem äußeren Magnetfeld die Matrix (2) mit zusätzlicher reiner Pufferlösung ausgespült und dabei das Eluat aufgefangen wird, wobei die Matrix (2) während des gesamten Vorgangs ständig von Pufferlösung bedeckt gehalten wird.

15. Verfahren nach einem der Ansprüche 11 bis 14,
wobei das aufgefangene Eluat zentrifugiert und mit dem abzentrifugierten biologischen Material ohne Flüssigkeitsphase des Eluates das Verfahren ein- oder mehrfach wiederholt wird.

## Claims

1. High gradient magnetic separation apparatus for the separation or purification of magnetic or magnetically labeled biological material having a magnet (7), a separation column (1) and a ferromagnetic matrix (2) arrangeable in an inner space of the separation column, as well as a storage container (11) containing a buffer solution for the equilibration of the separation column (1) and/or for the suspension of the biological material, wherein, during operation, a magnetic field generated by the magnet (7) can generate a high gradient magnetic field in the matrix (2) and buffer solution can flow through the separation column (1) from the liquid storage container (11),
**characterized in that**
the buffer solution encompasses a base solution and macromolecules which are able to saturate unspecific binding sites of the matrix (2), wherein the macromolecules comprise filamentary proteins, in particular gelatins.

2. An apparatus in accordance with claim 1,
wherein the buffer solution has a density which matches the density of the particles of the biological material to be separated so far that a force due to gravity acting on the particles is largely compensated and therefore, the particles are almost suspended in the buffer solution, and/or the buffer solution has a high viscosity which facilitates a laminar flow through the separation column (1) with a flow velocity suitable for the separation process.

3. An apparatus in accordance with claim 1 or 2,
wherein the magnet (7) is a permanent magnet or an electromagnet which is shaped so that the separation column (1) can be arranged in the particularly homogenous magnetic field generated by it, wherein the separation column (1) can selectively stand under the influence of the magnetic field or not by spatial separation and/or by switching it off.

4. An apparatus in accordance with one of the preceding claims,
wherein the matrix (2) is uncoated and/or has ordered or non-ordered filamentary, spherical or differently shaped material, in particular stainless, magnetic steel or steel wool.

5. An apparatus in accordance with any one of the preceding claims,
wherein the liquid storage container (11) is connected to the separation column (1) so that buffer solution can be introduced into the separation column (1) via a flow limitation device (8, 9) with an adjustable flow velocity or not, and/or wherein the separation column (1) has a flow limitation device (4, 5) which can influence the outflow out of, and the flow velocity inside the separation column.

6. An apparatus in accordance with any one of the preceding claims,
wherein the ionic strength of the base solution is adapted in dependence on the macromolecules so that aggregating effects of the macromolecules on the biological material are compensated, wherein the base solution has an isoosmolar concentration of the cations sodium, potassium, magnesium or calcium and of the anions chloride, phosphate, sulfate or carbonate, and is, in particular, a phosphate buffered saline or sucrose solution or a mixture thereof.

7. An apparatus in accordance with any one of the preceding claims,
wherein the macromolecules include natural or synthetic polyelectrolytes or polyampholytes, particularly synthetic polyelectrolyte or organic polyelectrolyte d-glucoronic acid, and/or wherein the macromolecules have an isoelectric point which leads, at the pH value of the base solution, to a charge which corresponds to the charge of the particles of the biological material to be separated, and/or wherein the macromolecules have a molecular weight of about 10,000 to about 100,000 kDa, particularly from about 30,000 to about 70,000 kDa.

8. An apparatus in accordance with any one of the preceding claims,
wherein the macromolecules include globular proteins, particularly albumins, bovine or human serumalbumin, ovoalbumin, lactoalbumin or plant albumins, β-lactoglobulin, κ-casein, histones, protamines, globulines, prolamines or glutelines with a concentration of about 3 % to about 7 %, particularly about 4 % to about 5 % in relation to the buffer solution.

9. An apparatus in accordance with any one of the preceding claims,
wherein the filamentary proteins include hydrolysed collagens in a concentration of about 0.3 to about 20 wt-%, particularly of about 1 to about 10 wt-%.

10. An apparatus in accordance with any of the preceding claims,
wherein the gelatins include bovine gelatins, porcine gelatins or teleosteangelatins and/or have a concentration of about 0.3 % to about 1.5 %, particularly from about 0.4 % to about 0.8 % and/or have a low gel strength of about 150 Bloom or less, particularly of about 75 Bloom or less.

11. A method for the separation or purification of intrinsically magnetic or preparatively magnetically labeled biological material by means of high gradient magnetic separation wherein the suspension with the biological material flows through a ferromagnetic matrix (2) arranged in an external magnetic field so that the material adheres to the matrix (2),
**characterized in that** the matrix (2) is uncoated, **in that** the biological material is suspended in a buffer solution with a base solution and macromolecules which saturate unspecific binding sites of the matrix (2) during flow-through the matrix, and **in that** the matrix (2) and a separation column (1) surrounding it is equilibrated before throughflow of the suspension by a pre-incubation with pure buffer solution, i.e. buffer solution containing no biological material, and over a sufficiently long period of time to saturate unspecific binding sites in the matrix (2).

12. A method in accordance with claim 11,
wherein the matrix (2) is equilibrated over a duration of about 3 to about 20 or about 5 to about 10 minutes, and wherein the matrix (2) is always kept covered by the buffer solution during equilibration.

13. A method in accordance with claim 11 or 12,
wherein for the separation of unwanted biological material the eluate, i.e. a liquid leaving the matrix (2) is captured after the throughflow, wherein after introduction of the suspension into the matrix (2) additional pure buffer solution is flowed through the matrix (2), with the magnetic field still activated until it is ensured that the suspension has completely left the matrix (2), and wherein the matrix (2) is always kept covered by the buffer solution during the throughflow.

14. A method in accordance with claim 11 or 12,
wherein, for the separation of wanted biological material, after introduction of the suspension into the matrix (2) with still activated magnetic field, additional pure buffer solution is flowed through the matrix (2) until it is ensured that the suspension has completely left the matrix (2), and subsequently the matrix (2) is washed with additional pure buffer solution with the external magnetic field deactivated by spatial separation or switch-off and wherein the eluate is captured, with the matrix (2) being always kept covered by the buffer solution during the entire process.

15. A method in accordance with any one of the claims 11 to 14,
wherein the eluate captured is centrifuged and the method is repeated with the centrifuged off biological material without the liquid phase of the eluate one or several times.

## Revendications

1. Appareil de séparation magnétique à haut gradient pour la séparation ou la purification de matériaux biologiques magnétiques ou marqués magnétiquement comprenant un aimant (7), une colonne de séparation (1) et une matrice ferromagnétique (2) susceptible d'être agencée dans un volume intérieur de la colonne de séparation, et comprenant un récipient de réserve (11) qui contient une solution tampon pour l'équilibrage de la colonne de séparation (1) et/ou pour la suspension du matériau biologique, dans lequel en fonctionnement un champ magnétique généré par l'aimant (7) peut engendrer dans la matrice (2) un champ magnétique à haut gradient et la solution tampon peut s'écouler hors du récipient de réserve (11) en traversant la colonne de séparation (1),
**caractérisé en ce que**
la solution tampon comprend une solution de base et des macromolécules qui sont capables de saturer des points de liaison non spécifiques de la matrice (2), lesdites macromolécules comprenant des protéines filamenteuses, en particulier les gélatines.

2. Appareil selon la revendication 1,
dans lequel la solution tampon a une densité qui correspond largement à la densité des particules à séparer du matériau biologique de telle façon qu'une force de gravité agissant sur les particules est essentiellement compensée et les particules sont par conséquent pratiquement en flottement dans la solution tampon, et/ou la solution tampon présente une forte viscosité qui permet un écoulement laminaire traversant la colonne de séparation (1) avec une vitesse d'écoulement convenable pour le processus de séparation.

3. Appareil selon la revendication 1 ou 2,
dans lequel l'aimant (7) est un aimant permanent ou un électroaimant, conformé de telle façon que la colonne de séparation (1) peut être agencée dans le champ magnétique, en particulier homogène, qu'elle engendre, et la colonne de séparation (1) peut ou non se trouver au choix sous l'action du champ magnétique par séparation géographique et/ou par coupure.

4. Appareil selon l'une des revendications précédentes,
dans lequel la matrice (2) est dépourvue de revêtement et/ou comprend un matériau ordonné ou non ordonné, filamenteux, sphérique ou d'une autre forme, en particulier de l'acier spécial ou de la laine d'acier inoxydable magnétique.

5. Appareil selon l'une des revendications précédentes,
dans lequel le récipient de réserve (11) est relié à la colonne de séparation (1) de telle façon que la solution tampon peut être ou non introduite via un système de limitation d'approvisionnement (8, 9) avec une vitesse d'écoulement réglable dans la colonne de séparation (1), et/ou la colonne de séparation (1) comprend un système de limitation d'écoulement traversant (4, 5) qui est capable d'influencer l'écoulement sortant de la colonne de séparation (1) et la vitesse d'écoulement dans la colonne de séparation (1).

6. Appareil selon l'une des revendications précédentes,
dans lequel l'intensité ionique de la solution de base est adaptée en fonction des macromolécules de telle façon que des effets agglutinants des macromolécules sur le matériau biologique sont compensés, et la solution de base présente une concentration isoosmolaire de cations sodium, potassium, magnésium ou calcium, et d'anions chlorure, phosphate, sulfate ou carbonate, en particulier une solution de sel ou de saccharose avec tampon de phosphate, ou un mélange de telles solutions.

7. Appareil selon l'une des revendications précédentes,
dans lequel les macromolécules comprennent des polyélectrolytes ou polyampholytes naturels ou synthétiques, en particulier un polyélectrolyte synthétique ou un polyélectrolyte organique de acide glucuronique D, et/ou dans lequel les macromolécules présentent un point isoélectrique qui, lorsque la solution de base présente un pH, mène à une charge qui correspond à la charge des particules à séparer du matériau biologique, et/ou dans lequel les macromolécules présentent un poids moléculaire d'environ 10 000 à environ 100 000 kDa, en particulier d'environ 30 000 à environ 70 000 kDa.

8. Appareil selon l'une des revendications précédentes,
dans lequel les macromolécules comprennent des protéines globulaires, en particulier de l'albumine, de l'albumine de sérum bovin ou humain, ovalbumine, lactalbumine ou albumine végétale, β-lactoglobuline, κ-caséine, histone, protamine, globuline, prolamine ou gluteline avec une concentration d'environ 3 % à environ 7 %, en particulier environ 4 % à environ 5 % par référence à la solution tampon.

9. Appareil selon l'une des revendications précédentes,
dans lequel les protéines filamenteuses comprennent des collagènes hydrolysés dans une concentration d'environ 0,3 à environ 20 % en bois, en particulier d'environ 1 % à environ 10 % en poids.

10. Appareil selon l'une des revendications précédentes,
dans lequel les gélatines, gélatines bovines, gélatines porcines ou gélatines de téléostélien présentent une concentration d'environ 0,3 % à environ 1,5 %, en particulier environ 0,4 % à environ 0,8 % et/ou présentent un pouvoir de gélification inférieur d'environ 150 Bloom ou moins, en particulier environ 75 Bloom ou moins.

11. Procédé pour la séparation ou la purification de matériaux biologiques magnétiques ou marqués magnétiquement de façon préparatoire, au moyen d'une séparation magnétique à haut gradient, dans lequel une suspension avec le matériau biologique s'écoule en traversant une matrice ferromagnétique (2) agencée dans un champ magnétique extérieur de telle façon que le matériau adhère à la matrice (2), **caractérisé en ce que**
la matrice (2) est dépourvue de revêtement,
**en ce que** le matériau biologique est mis en suspension dans une solution tampon avec une solution de base et des macromolécules qui, lors de la traversée de la matrice, sature des points de liaison non spécifiques de la matrice (2), et
**en ce que** la matrice (2) et une colonne de séparation (1) qui l'entoure sont équilibrées, avant écoulement de la suspension, par une incubation préliminaire avec une solution tampon pure, donc qui ne contient aucun matériau biologique, sur une période suffisamment longue, afin de saturer des points de liaison non spécifiques dans la matrice (2).

12. Procédé selon la revendication 11,
dans lequel la matrice (2) est équilibrée sur une durée d'environ 3 à environ 20, ou environ 5 à environ 10 minutes, et dans lequel la matrice (2) est maintenue couverte en permanence par la solution tampon pendant l'équilibrage.

13. Procédé selon la revendication 11 ou 12,
dans lequel pour séparer un matériau biologique indésirable, le produit élué, donc un fluide qui quitte la matrice (2), est capté après la traversée, dans lequel après introduction de la suspension dans la matrice (2) alors que le champ magnétique est toujours activé, la matrice (2) est traversée par une solution tampon pure additionnelle aussi longtemps que l'on assure que la suspension à entièrement quitté la matrice (2), et dans lequel la matrice (2) est maintenue couverte en permanence par la solution tampon pendant la traversée.

14. Procédé selon la revendication 11 ou 12,
dans lequel pour purifier des matériaux biologiques souhaités, après introduction de la suspension dans la matrice (2) alors que le champ magnétique est toujours activé, la matrice (2) est traversée par une solution tampon pure additionnelle aussi longtemps que l'on assure que la suspension a entièrement quitté la matrice (2), et ensuite alors que le champ magnétique extérieur est désactivé soit par séparation géographique soit par coupure, la matrice (2) est rincée avec une solution tampon pure additionnelle et le produit élué est ici capté, et la matrice (2) est maintenue couverte en permanence par la solution tampon pendant la totalité du processus.

15. Procédé selon l'une des revendications 11 à 14,
dans lequel le produit élué capté est centrifugé et on répète le processus une ou plusieurs fois avec le matériau biologique centrifugé dépourvu de la phase liquide du produit élué.
